# EUROPEAN PATENT APPLICATION

(11) **EP 4 768 075 A1**
(43) Date of publication of application: **01.07.2026**
(21) Application number: 25227550.8
(22) Date of filing: 30.12.2025
(51) Int. Cl.: A61N 5/10

(54) **RADIOTHERAPY CONTROL AND RADIOTHERAPY PLAN GENERATION METHODS, DEVICE, SYSTEM AND MEDIUM**

(30) Priority: 26.12.2024 CN 202411942592
(71) Applicant: Our United Corporation, Xi'an, Shaanxi 710018 (CN)
(72) Inventor: LIU, Haifeng, Xi'an Economic & Technological Development Zone (CN); YAN, Hao, Xi'an Economic & Technological Development Zone (CN); ZHANG, Zhongyuan, Xi'an Economic & Technological Development Zone (CN)
(74) Representative: Murgitroyd & Company

(57) **Abstract**

The present disclosure provides radiotherapy control and radiotherapy plan generation methods, device, and system, and a medium. The method includes: controlling a diagnosis-treatment integrated couch to move a target object to a diagnostic device, so as to acquire a diagnostic image of the target object through the diagnostic device; and controlling the diagnosis-treatment integrated couch to move the target object from the diagnostic device to a radiotherapy device, and synchronously triggering generation of a radiotherapy plan for the target object based on the diagnostic image.

## Description

### TECHNICAL FIELD

The present disclosure relates to the field of medical technologies, particularly to the field of radiation treatment technologies, and specifically to radiotherapy control and radiotherapy plan generation methods, device, and system, and a medium.

### BACKGROUND

At present, when radiation treatment is performed on a patient, it is usually necessary that positioning image scanning is first performed by a magnetic resonance imaging device to obtain a scan image of the patient, and then, a treatment plan is determined according to the scan image, and the radiation treatment is performed according to the treatment plan through a radiation radiotherapy device.

However, since the magnetic resonance imaging device and the radiation radiotherapy device are usually located in two different rooms (for example, the magnetic resonance imaging device is usually deployed in a imaging room, while the radiation radiotherapy device is usually deployed in a treatment room), the patient still needs to go to the treatment room for the radiation treatment after the positioning image scan is performed on the patient. In this way, not only does the patient need to move multiple times, but also the patient needs to be set up again before the radiation radiotherapy device is used for the treatment, which is time-consuming, laborious, inefficient and to some extent reduces the treatment effect.

### SUMMARY

The present disclosure provides radiotherapy control and radiotherapy plan generation methods, device, and system, and a medium, which may improve the efficiency of the radiotherapy plan generation.

In a first aspect, the present disclosure provides a radiotherapy control method, including: controlling a diagnosis-treatment integrated couch to move a target object to a diagnostic device, so as to acquire a diagnostic image of the target object through the diagnostic device; and controlling the diagnosis-treatment integrated couch to move the target object from the diagnostic device to a radiotherapy device, and synchronously triggering generation of a radiotherapy plan for the target object based on the diagnostic image.

In a second aspect, it provides a radiotherapy plan generation method, performed by a TPS server, and including: receiving a radiotherapy plan generation instruction sent from a control device; the radiotherapy plan generation instruction is triggered synchronously when the control device controls a diagnosis-treatment integrated couch to move a target object from a diagnostic device to a radiotherapy device; and in response to the radiotherapy plan generation instruction, generating a radiotherapy plan for the target object based on a diagnostic image; the diagnostic image is acquired by the diagnostic device, through the control device controlling the diagnosis-treatment integrated couch to move the target object to the diagnostic device, before triggering the radiotherapy plan generation instruction.

In a third aspect, the present disclosure also provides an electronic device, and the electronic device includes: a processor, and a memory configured to store instructions executable by the processor; where the processor is configured to execute the instructions to implement any radiotherapy control method in the above first aspect or any radiotherapy plan generation method in the above second aspect.

In a fourth aspect, the present disclosure further provides a non-volatile storage medium, a computer program is stored on the storage medium, and the computer program, when read and executed, implements any radiotherapy control method in the above first aspect or any radiotherapy plan generation method in the above second aspect.

In a fifth aspect, the present disclosure also provides a radiotherapy system, including: a control device, configured to perform any radiotherapy control method in the first aspect; and a TPS server, configured to perform any radiotherapy plan generation method in the second aspect.

In the radiotherapy control method and the radiotherapy plan generation method provided in the present disclosure, the control device may first control a diagnosis-treatment integrated couch to move a target object to a diagnostic device, so as to acquire a diagnostic image of the target object through the diagnostic device. Then, the control device may control the diagnosis-treatment integrated couch to move the target object from the diagnostic device to a radiotherapy device, and synchronously trigger the TPS server to generate a radiotherapy plan for the target object based on the diagnostic image. After receiving a radiotherapy plan generation instruction sent from the control device, the TPS server may generate a radiotherapy plan for the target object based on the diagnostic image in response to the radiotherapy plan generation instruction.

As can be seen from the above, during controlling the diagnosis-treatment integrated couch to move the target object from the diagnostic device to the radiotherapy device, the control device may synchronously trigger the TPS server to generate the radiotherapy plan for the target object, thereby saving the time for the target object to wait for the generation of the radiotherapy plan, improving the generation efficiency of the radiotherapy plan, and then, improving the efficiency of the radiation treatment.

Moreover, since the diagnostic device and the radiotherapy device are in the same space, and the target object may be moved between the diagnostic device and the radiotherapy device through the diagnosis-treatment integrated couch. In this way, by controlling the diagnosis-treatment integrated couch, the target object may be moved to the radiotherapy device (i.e., a position for the radiation treatment) for the treatment, and there is no need for the target object to go to the treatment room for the treatment, and no need to set up the target object again, which further improves the efficiency and effect of the treatment.

### BRIEF DESCRIPTION OF THE DRAWINGS

The drawings are used for a better understanding of the present solution and do not constitute a limitation on the present disclosure.
FIG. 1 is a schematic diagram of a scenario of a radiotherapy system provided in the embodiments of the present disclosure.
FIG. 2 is a schematic flowchart of a radiotherapy control method provided in the embodiments of the present disclosure.
FIG. 3 is a schematic flowchart of another radiotherapy control method provided in the embodiments of the present disclosure.
FIG. 4 is a schematic diagram of a scenario of a radiotherapy control method provided in the embodiments of the present disclosure.
FIG. 5 is a schematic flowchart of another radiotherapy control method provided in the embodiments of the present disclosure.
FIG. 6 is a schematic flowchart of another radiotherapy control method provided in the embodiments of the present disclosure.
FIG. 7 is a schematic flowchart of a radiotherapy plan generation method provided in the embodiments of the present disclosure.
FIG. 8 is a schematic flowchart of another radiotherapy plan generation method provided in the embodiments of the present disclosure.
FIG. 9 is a schematic block diagram of an electronic device provided in the embodiments of the present disclosure.

### DETAILED DESCRIPTION

The following will describe clearly and completely the technical solutions of the embodiments of the present disclosure in conjunction with the drawings in the embodiments of the present disclosure, and obviously, the described embodiments are only some of the embodiments of the present disclosure, not all of the embodiments. All other embodiments obtained by those skilled in the art without making creative efforts based on the embodiments in the present disclosure fall within the protection scope of the present disclosure.

In the description of the present disclosure, the word "exemplary/exemplarily" is used to represent "as an example, illustration or description". Any embodiment described with "exemplary/exemplarily" in the present disclosure is not necessarily construed as being more preferred or advantageous than other embodiments. To enable any skilled in the art to implement and use the present disclosure, the following description is given. In the following description, the details are listed for the purpose of explanation. It should be understood that those skilled in the art can recognize that the present disclosure may also be implemented without using these specific details. In other instances, the publicly known structures and processes will not be described in detail, so as to avoid unnecessary details to make the description in the present disclosure obscure. Therefore, the present disclosure is not intended to be limited to the shown embodiments, but is consistent with the broadest scope that conforms to the principles and features disclosed in the present disclosure.

It should be noted that since the radiotherapy control method provided in the embodiments of the present disclosure is performed in a control device, and processes objects of the control device all exist in the form of data or information, for example, time, which is essentially time information, it can be understood that in subsequent embodiments, if dimensions, numbers, positions, etc., are mentioned, they all exist as corresponding data, so as to facilitate processing by the control device, and the specific details will not be repeated here.

In a procedure of a tumor radiation treatment, multi-fraction of radiation treatment are generally required. In a complete treatment period, there may be setting up errors, or it is very difficult to ensure that radiation beams are completely aimed at a target region for irradiation due to the influence of factors such as the shape, size, position changes of the tumor itself, and human breathing, etc., which will result in insufficient irradiation on the tumor tissue by radiation, leading to recurrence, while the surrounding normal tissues will also be unnecessarily irradiated.

In order to solve these problems, the image-guided radiotherapy technology (Image Guided Radiotherapy, IGRT) comes into being. The image-guided radiotherapy technology may be image-guided based on the imaging of an imaging device. The early online two-dimensional X-ray cross (Digitally Reconstructed Radiography, DR) and cone-beam computed tomography (Cone - Beam Computed Tomography, CBCT) are relatively advanced, and widely apply imaging technologies. However, images generated by CBCT often lack soft tissue contrast ratio and a precise boundary between a normal organ and a tumor cannot be accurately identified, and therefore, the main problem to be solved is setting up errors in fractions of treatment. However, it is powerless to solve the problems caused by changes in the size, shape and position of the tumor in the treatment cycle. In this way, the online electronic computed tomography (Computed Tomography, CT) and magnetic resonance imaging (Magnetic Resonance Imaging, MRI) image-guided technologies are introduced, so as to not only solve the problems of setting up errors, but also adjust the plan to solve the problem caused by changes in the size, shape, position of the tumor, etc., thereby implementing adaptive radiotherapy based on the position and shape of the tumor. If the breath holding, respiratory gating technology, four-dimensional radiotherapy technology and real-time tracking technology are combined, the treatment target region may be further reduced, so as to achieve a better radiotherapy effect.

An MRI image provides superior imaging of a high-definition contrast ratio of a soft tissue, which may clearly distinguish a boundary between the tumor and adjacent normal tissues, and may identify tumor-related physiological and anatomical changes (such as intestinal peristalsis, changes in respiration or tumor size, shape, and position) during radiotherapy. This enables radiation oncologists to precisely grasp the dose field changes caused by these structural changes in the treatment process, and quickly make an adaptive adjustment, and due to the non-radioactive property of MR imaging, the adaptive radiation treatment based on the MRI image may also be implemented by evaluating the radiotherapy effect through functional imaging.

However, since the magnetic resonance imaging device (i.e., MRI device) and the radiation radiotherapy device are usually located in two different rooms (for example, the magnetic resonance imaging device is usually deployed in a imaging room, while the radiation radiotherapy device is usually deployed in a treatment room), the patient still needs to go to the treatment room for the radiation treatment after the positioning image scan is performed on the patient. In this way, not only does the patient need to move multiple times, but also the patient needs to be set up again before the radiation radiotherapy device is used for the treatment, which is time-consuming, laborious, inefficient and to some extent reduces the treatment effect.

Based on the above technical problems, the embodiments of the present disclosure provide a radiotherapy control method and a radiotherapy plan generation method, where the control device may first control a diagnosis-treatment integrated couch to move a target object to a diagnostic device, so as to acquire a diagnostic image of the target object through the diagnostic device. Then, the control device may control the diagnosis-treatment integrated couch to move the target object from the diagnostic device to a radiotherapy device, and synchronously trigger a radiation treatment planning system (Treatment Planning System, TPS) server to generate a radiotherapy plan for the target object based on the diagnostic image. After receiving a radiotherapy plan generation instruction sent from the control device, the TPS server may generate a radiotherapy plan for the target object based on the diagnostic image in response to the radiotherapy plan generation instruction.

As can be seen from the above, in the process where the control device controls the diagnosis-treatment integrated couch to move the target object from the diagnostic device to the radiotherapy device, it may synchronously trigger the TPS server to generate the radiotherapy plan for the target object, thereby saving the time for the target object to wait for the generation of the radiotherapy plan, improving the generation efficiency of the radiotherapy plan, and then improving the efficiency of the radiation treatment.

Moreover, since the diagnostic device and the radiotherapy device are in the same space, the target object may be moved between the diagnostic device and the radiotherapy device through the diagnosis-treatment integrated couch. In this way, by controlling the diagnosis-treatment integrated couch, the target object may be moved to the radiotherapy device (i.e., a position for the radiation treatment) for the treatment, and there is no need for the target object to go to the treatment room for the treatment, and no need to set up the target object again, which further improves the efficiency and effect of the treatment.

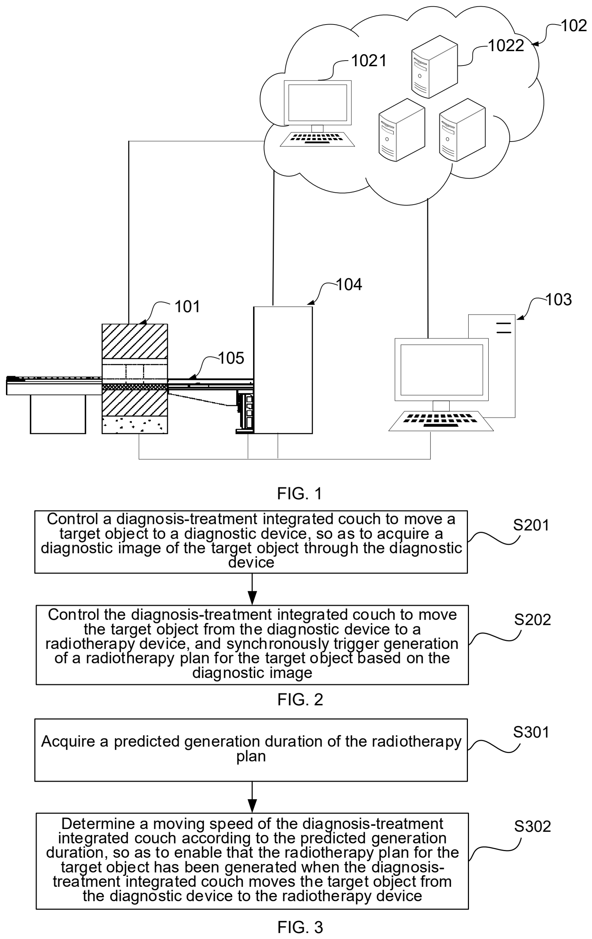

The above radiotherapy control method and radiotherapy plan generation method may be applied to a radiotherapy system. FIG. 1 is a schematic diagram of a scenario of a radiotherapy system provided in the embodiments of the present disclosure, and the radiotherapy system may include: a diagnostic device (also referred to as image acquisition device) 101, a radiotherapy planning device 102, a control device (also referred to as a control computer device) 103, a radiotherapy device (also referred to as a radiation treatment device) 104 and a diagnosis-treatment integrated couch (also referred to as a support apparatus) 105.

The diagnostic device 101 is a device configured to acquire images of a tumor site (i.e., a target region) and surrounding normal tissues of a target object (for example, a patient to be treated, an experimental object, a model, etc.). In some embodiments, the diagnostic device 101 may be at least one of a computed tomography (Computed Tomography, CT) device or an emission computed tomography (Emission Computed Tomography, ECT) device, a magnetic resonance imaging (Magnetic Resonance Imaging, MRI) device, a positron emission tomography (PET) device, and an ultrasound examination device.

In the embodiments of the present disclosure, the diagnostic device 101 is used to acquire a diagnostic image of the target object.

In some embodiments, in a case where the target object is injected with a tracer, the diagnostic device includes at least one of: a CT device, an MRI device, and a PET device.

In some embodiments, the above-mentioned tracer may be a sugar tracer.

The radiotherapy device 104 is a device configured for radiation treatment on the target object. In some embodiments, the radiotherapy device 104 may include a gantry, a treatment head and an image-guided apparatus (since FIG. 1 is a side view of the radiotherapy device 104, a structural schematic diagram of the gantry, treatment head and image-guided apparatus has not been shown in FIG. 1).

The gantry may be a rotatable gantry. The treatment head may be set on the gantry and is used to emit a radiation beam, such as gamma rays, MV-level X-rays, proton rays, etc., so as to radiate an object to be irradiated. For example, the treatment head may be any two of a gamma knife treatment head for rotating focused radiation treatment, an accelerator treatment head for adaptive intensity-modulated radiation treatment, or other radiation treatment heads. The image-guided apparatus is configured to set up the target object and perform real-time image guidance in radiation treatment, and exemplarily, the image-guided apparatus includes a bulb tube and a detector, the detector may receive an imaging beam emitted from the bulb tube that passes through the target object and generate a projected image.

A diagnosis-treatment integrated couch 105 is configured to support and move the target object, and may be a treatment bed.

In some embodiments, in a case where the target object is on the diagnosis-treatment integrated couch 105, the rotation of the gantry may drive the treatment head to perform 360-degree irradiation around the target object, thereby completing radiation treatment.

In the embodiments of the present disclosure, the diagnostic device 101 and the radiotherapy device 104 are in the same space (for example, the same room). The diagnosis-treatment integrated couch 105 may be deployed between the diagnostic device 101 and the radiotherapy device 104, and may also be moved between the diagnostic device 101 and the radiotherapy device 104.

When the diagnosis-treatment integrated couch 105 is moved into the diagnostic range of the diagnostic device 101, the diagnostic device 101 may perform image acquisition on the target object on the diagnosis-treatment integrated couch 105 to obtain the diagnostic image of the target object.

When the diagnosis-treatment integrated couch 105 is moved into the treatment range of the radiotherapy device 104, the radiotherapy device 104 may perform radiotherapy on the target object on the diagnosis-treatment integrated couch 105.

The radiotherapy planning device 102 is a device configured to acquire the diagnostic image of the target object from the diagnostic device 101 to formulate, optimize and evaluate the radiotherapy plan. The radiotherapy planning device 102 may operate with a radiation treatment planning system (Treatment Planning System, TPS), and the radiation treatment planning system provides functions of formulating, optimizing and evaluating the radiotherapy plan, for example, an RT pro TPS system.

In some embodiments, the radiotherapy planning device 102 may include a TPS client 1021 and a TPS server 1022.

The TPS client 1021 may be at least one of devices such as a smart phone, a smart watch, a desktop computer, a handheld computer, a virtual reality terminal, an augmented reality terminal, a wireless terminal and a laptop portable computer, etc. For example, in some embodiments, a user may trigger, through the radiation treatment planning system operating on the TPS server 1022, by the TPS client 1021, the TPS server 1022 to perform an adaptive radiotherapy planning optimization process, and display an optimized radiotherapy plan. In this way, user's time may be effectively saved, and the optimized radiotherapy plan may be presented more intuitively, which is conducive for the user to evaluate the radiotherapy plan.

The TPS server 1022 may be at least one of an independent physical server, or a server cluster composed of multiple physical servers, or a distributed file system, or a cloud server that provides a basic cloud computing service such as a cloud service, a cloud database, cloud computing, a cloud function, a cloud storage, a network service, a cloud communication, a middleware service, a domain name service, a security service, a content distribution network, and big data or an artificial intelligence platform, which are not limited in the embodiments of the present disclosure. In some embodiments, the number of the above-mentioned TPS servers 1022 can be more or less, which is not limited in the embodiments of the present disclosure. Of course, the TPS server 1022 can also include other functions, so as to provide more comprehensive and diverse services. In some embodiments, the TPS server 1022 is configured to provide a back-end service for the above-mentioned TPS client 1021, for example, so as to perform an adaptive radiotherapy plan optimization process.

In an embodiment of the present disclosure, the TPS server 1022 in the radiotherapy planning device 102 may receive a radiotherapy planning generation instruction sent from the control device 103, and generate a radiotherapy plan for the target object based on the diagnostic image obtained from the diagnostic device 101, in response to the radiotherapy planning generation instruction.

That is, in the process where the diagnosis-treatment integrated couch 105 is moved between the diagnostic device 101 and the radiotherapy device 104, the TPS server 1022 in the radiotherapy planning device 102 may generate the radiotherapy plan for the target object during the process.

In some embodiments, the control device 103 may directly send the radiotherapy plan generation instruction to the TPS server 1022, or send the radiotherapy plan generation instruction to the TPS server 1022 through the TPS client 1021 in the radiotherapy plan device 102, which is not limited in the embodiments of the present disclosure.

The control device 103 is a device configured to control the radiotherapy device 104 to perform the radiotherapy plan. In some embodiments, the control device 103 may include an upper machine and a lower machine, the upper machine is configured to interact with the user, and the lower machine is configured to control the movement of moving components in the radiotherapy device 104, and the upper machine may be at least one of devices such as a smart phone, a smart watch, a desktop computer, a handheld computer, a virtual reality terminal, an augmented reality terminal, a wireless terminal and a laptop portable computer, etc., and/or a server device. The lower machine may be a programmable logic controller (Programmable Logic Controller, PLC) and other control devices.

In the embodiments of the present disclosure, the control device 103 is configured to perform the radiotherapy control method provided in the embodiments of the present disclosure, and for example, it controls the diagnosis-treatment integrated couch 105 to move the target object to the diagnostic device 101 to acquire the diagnostic image of the target object through the diagnostic device 101, and controls the diagnosis-treatment integrated couch 105 to move the target object from the diagnostic device 101 to the radiotherapy device 104, and synchronously triggers the TPS server 1022 in the radiotherapy planning device 102 to generate the radiotherapy plan for the target object based on the diagnostic image.

In some embodiments, the control device 103 may also display and control a state (for example, on/off state, etc.) and a dynamic (for example, a current moving position, etc.) of the diagnosis-treatment integrated couch 105.

In some embodiments, an entity of the control device 103 may be a terminal, or a server with a display, which is not limited in the embodiments of the present disclosure.

In some embodiments, the above-mentioned terminal may be at least one of devices such as a smart phone, a smart watch, a desktop computer, a handheld computer, a virtual reality terminal, an augmented reality terminal, a wireless terminal and a laptop portable computer, etc.

In some embodiments, the above-mentioned server may be at least one of an independent physical server, or a server cluster composed of multiple physical servers, or a distributed file system, or a cloud server that provides a basic cloud computing service such as a cloud service, a cloud database, cloud computing, a cloud function, a cloud storage, a network service, a cloud communication, a middleware service, a domain name service, a security service, a content distribution network, and big data or an artificial intelligence platform, which are not limited in the embodiments of the present disclosure. In some embodiments, the number of the above-mentioned servers can be more or less, which is not limited in the embodiments of the present disclosure. Of course, the server can also include other functions, so as to provide more comprehensive and diverse services.

Furthermore, in some embodiments, the control device 103 includes a processor, and the processor is configured to implement the radiotherapy control method provided in the embodiments of the present disclosure.

In some embodiments, the control device 103 may be deployed in the same space as the diagnostic device 101 and the radiotherapy device 104, for example, in the same room, or in a different space from the diagnostic device 101 and the radiotherapy device 104, or deployed in the cloud synchronously with the TPS server 1022, which is not limited in the embodiments of the present disclosure.

The following introduces the radiotherapy control method provided in the embodiments of the present disclosure, based on the radiotherapy system shown in FIG. 1.

The radiotherapy control method provided in the embodiments of the present disclosure is performed by the control device 103 in FIG. 1. FIG. 2 shows a schematic flowchart of a radiotherapy control method provided in the embodiments of the present disclosure. As shown in FIG. 2, the radiotherapy control method includes: S201 to S202.

S201: the control device controls a diagnosis-treatment integrated couch to move a target object to a diagnostic device, so as to acquire a diagnostic image of the target object through the diagnostic device.

In some embodiments, the control device may pre-store position information of a diagnostic range of the diagnostic device. In response to receiving an indication for moving the diagnosis-treatment integrated couch into the diagnostic range of the diagnostic device, the control device may control the diagnosis-treatment integrated couch to move the target object to the diagnostic device, according to the pre-stored position information of the diagnostic range of the diagnostic device.

In some embodiments, the control device may also acquire a relative position relationship between the diagnostic device and the diagnosis-treatment integrated couch. In response to receiving an indication for moving the diagnosis-treatment integrated couch into the diagnostic range of the diagnostic device, the control device may control the diagnosis-treatment integrated couch to move the target object to the diagnostic device, according to the relative position relationship between the diagnostic device and the diagnosis-treatment integrated couch.

In some embodiments, the therapist may control the diagnosis-treatment integrated couch to move the target object to the diagnostic device, through the control device by manually operating.

In some embodiments, when the diagnostic device acquires the diagnostic image of the target object, it may automatically acquire the diagnostic image of the target object while it detects that the diagnosis-treatment integrated couch has moved into the diagnostic range of the diagnostic device; or it may acquire the diagnostic image of the target object, by receiving an instruction from a manual operation, while it detects that the diagnosis-treatment integrated couch is moved into the diagnostic range of the diagnostic device. Of course, the diagnostic device may also receive an image acquisition instruction sent from the control device to acquire the diagnostic image of the target object, when the diagnosis-treatment integrated couch moves the target object into the diagnostic range of the diagnostic device.

After acquiring the diagnostic image of the target object, the diagnostic device may send it to the TPS server through a formulated address.

In some embodiments, the target object needs to be pre-positioned before undergoing radiotherapy on the diagnostic device. The pre-positioned position may be the same as or have a certain position relationship with a position that an initial radiotherapy plan is formulated. When performing pre-positioning, a fixing accessory, for example, a fixing headrest, a fixing bag, etc., may be added to prevent the target object from moving actively.

S202, the control device controls the diagnosis-treatment integrated couch to move the target object from the diagnostic device to a radiotherapy device, and synchronously triggers generation of a radiotherapy plan for the target object based on the diagnostic image.

After the diagnostic device acquires the diagnostic image of the target object, in order to improve the generation efficiency of the radiotherapy plan, the control device may control the diagnosis-treatment integrated couch to move the target object from the diagnostic device to the radiotherapy device, and synchronously trigger generation of the radiotherapy plan for the target object based on the diagnostic image.

It should be noted that the control device controlling the diagnosis-treatment integrated couch to move the target object from the diagnostic device to the radiotherapy device, here, means controlling the diagnosis-treatment integrated couch to move the target object from the diagnostic device to a treatment position of the radiotherapy device, or to a preset position that has a certain position relationship with the treatment position, which is then conducive for the radiotherapy device to perform radiotherapy on the target object.

In some embodiments, the above-mentioned treatment position and preset position may be pre-stored in the control device, or the control device may acquire them in real time from a server that stores the above-mentioned treatment position and preset position, after the diagnostic device acquires the diagnostic image of the target object.

In some embodiments, during controlling the diagnosis-treatment integrated couch to move the target object from the diagnostic device to the radiotherapy device, the control device may first move the target object from the diagnostic device to a transfer preparation position, and then from the transfer preparation position to the radiotherapy device; or may directly move it from the diagnostic device to the radiotherapy device.

In some embodiments, during the control device controlling the diagnosis-treatment integrated couch to move the target object from the diagnostic device to the radiotherapy device, the movement speed of the diagnosis-treatment integrated couch should not be too fast, which not only avoids the displacement of the target object due to too fast speed, but also avoids that the generation of the radiotherapy plan is not completed after the target object is moved to the radiotherapy device.

In some embodiments, the control device may synchronously trigger generation of the radiotherapy plan for the target object during the movement of the diagnosis-treatment integrated couch, or synchronously trigger generation of the radiotherapy plan for the target object at a beginning of the movement of the diagnosis-treatment integrated couch, or trigger generation of the radiotherapy plan for the target object after acquiring the diagnostic image. That is, the generation of the radiotherapy plan for the target object is completed, whenever the diagnosis-treatment integrated couch is controlled to move the target object from the diagnostic device to the radiotherapy device. In this case, the method of the control device controlling the diagnosis-treatment integrated couch to move the target object from the diagnostic device to a radiotherapy device, and synchronously triggering generation of a radiotherapy plan for the target object based on the diagnostic image, specifically includes:
controlling the diagnosis-treatment integrated couch to move the target object from the diagnostic device to the radiotherapy device, and synchronously triggering generation of the radiotherapy plan for the target object based on the diagnostic image during the movement of the diagnosis-treatment integrated couch.

Specifically, after the diagnostic device acquires the diagnostic image of the target object, the control device may first control the diagnosis-treatment integrated couch to move the target object from the diagnostic device to the radiotherapy device. During the movement of the diagnosis-treatment integrated couch (for example, after the control device controls the movement of the diagnosis-treatment integrated couch for 1 second or 2 seconds), the control device may synchronously trigger generation of the radiotherapy plan for the target object based on the diagnostic image. That is, after the diagnostic device acquires the diagnostic image of the target object, the control device first controls the diagnosis-treatment integrated couch to move, and then triggers generation of the radiotherapy plan for the target object.

Alternatively, the control device synchronously triggers generation of the radiotherapy plan for the target object based on the diagnostic image at a beginning of the diagnosis-treatment integrated couch to move the target object from the diagnostic device to the radiotherapy device.

That is, after the diagnostic device acquires the diagnostic image of the target object, the control device, at the same time of controlling the movement of the diagnosis-treatment integrated couch, synchronously triggers generation of the radiotherapy plan for the target object.

Alternatively, the control device synchronously triggers generation of the radiotherapy plan for the target object based on the diagnostic image at a moment when the diagnostic image is acquired, and controls the diagnosis-treatment integrated couch to move the target object from the diagnostic device to the radiotherapy device after the diagnostic device acquires the diagnostic image.

Specifically, the control device may synchronously trigger generation of the radiotherapy plan for the target object based on the diagnostic image at a moment when the diagnostic device acquires the diagnostic image of the target object, and control the diagnosis-treatment integrated couch to move the target object from the diagnostic device to the radiotherapy device after the diagnostic device acquires the diagnostic image (for example, 1 second or 2 seconds after acquiring the diagnostic image). That is, the control device first triggers generation of the radiotherapy plan for the target object, and after the diagnostic device acquires the diagnostic image of the target object, controls the movement of the diagnosis-treatment integrated couch.

To sum up, regardless of when the control device controls the diagnosis-treatment integrated couch to move the target object from the diagnostic device to the radiotherapy device, and regardless of when the control device synchronously triggers generation of the radiotherapy plan for the target object based on the diagnostic image, as long as the radiotherapy plan for the target object has been generated when the diagnosis-treatment integrated couch is controlled to move the target object from the diagnostic device to the radiotherapy device.

In some embodiments, when the radiotherapy device performs radiotherapy on the target object, the target object may also be positioned through an image-guided apparatus in the radiotherapy device. For example, the image-guided device may acquire a real-time KV projection of the target object, and position the target object (for example, reset the target region, etc.) according to a comparison result between the real-time KV projection and a DRR (Digitally Reconstructed Radiograph) image of the diagnostic image at a corresponding angle.

In some embodiments, the above-mentioned diagnostic image may be a DRR image that is converted from an MR Format to a CT format by imaging software.

In some embodiments, the above-mentioned comparison result between the images may be a comparison result for a certain section (e.g., sagittal plane, coronal plane) containing the target region in the target object.

In some embodiments, the control device is generally a simple control apparatus with a relatively single function, and therefore, generating the radiotherapy plan for the target object is generally performed by the TPS server. Of course, after integrating the radiotherapy plan generation function into the control device, the control device may also directly generate the radiotherapy plan for the target object.

That is, the control device may directly generate the radiotherapy plan for the target object, or may trigger generation of the radiotherapy plan for the target object based on the diagnostic image through the TPS server. In this case, the control device triggering generation of the radiotherapy plan for the target object based on the diagnostic image, includes:
in a case where the control device is integrated with the radiotherapy plan generation function, triggering the control device to generate the radiotherapy plan for the target object based on the diagnostic image. Alternatively, in a case where the control device is not integrated with the radiotherapy plan generation function, the control device sends a radiotherapy plan generation instruction to the TPS server, so as to trigger the TPS server to generate the radiotherapy plan for the target object based on the diagnostic image.

In some embodiments, in a case where the control device is not integrated with the radiotherapy plan generation function, the control device may directly send a radiotherapy plan generation instruction to the TPS server, or send a radiotherapy plan generation instruction to the TPS server through the TPS client, so as to trigger the TPS server to generate the radiotherapy plan for the target object based on the diagnostic image.

In some embodiments, the control device may adjust the moving speed of the diagnosis-treatment integrated couch according to a predicted generation duration of the radiotherapy plan. In this case, as shown in FIG. 3, the radiotherapy control method provided in the embodiments of the present disclosure also includes:
S301, the control device acquires a predicted generation duration of the radiotherapy plan.

In some embodiments, the predicted generation duration is predicted according to a generation duration of a radiotherapy plan for a reference image. The reference image is an image whose similarity to the diagnostic image is greater than a preset similarity. For example, the reference image may be a diagnostic image of a reference object with a similar feature (such as age, body size, and disease condition, etc.) to the target object. That is, the predicted generation duration of the radiotherapy plan may be generated according to a completed radiotherapy plan (i.e., the radiotherapy plan of the reference image).

In some embodiments, the predicted generation duration may also be set according to the therapist's experience or predicted through an artificial intelligence model, which is not limited in the embodiments of the present disclosure.

In some embodiments, the predicted generation duration may be pre-stored in the control device, or in a cloud server communicating with the control device, which is not limited in the embodiments of the present disclosure.

S302, the control device determines a moving speed of the diagnosis-treatment integrated couch according to the predicted generation duration, so as to enable that the radiotherapy plan for the target object has been generated when the diagnosis-treatment integrated couch moves the target object from the diagnostic device to the radiotherapy device.

Specifically, the control device may pre-store a position relationship between the diagnostic device and the radiotherapy device. After acquiring the predicted generation duration, the control device may determine a distance between the diagnostic device and the radiotherapy device according to the position relationship between the diagnostic device and the radiotherapy device, and determine the moving speed of the diagnosis-treatment integrated couch according to a quotient of the distance and the predicted generation duration.

Of course, the moving speed may be less than or equal to the quotient of the distance and the predicted generation duration, as long as it is ensured that the radiotherapy plan for the target object has been generated when the diagnosis-treatment integrated couch moves the target object from the diagnostic device to the radiotherapy device, which may also avoid the displacement of the target object due to the too fast speed.

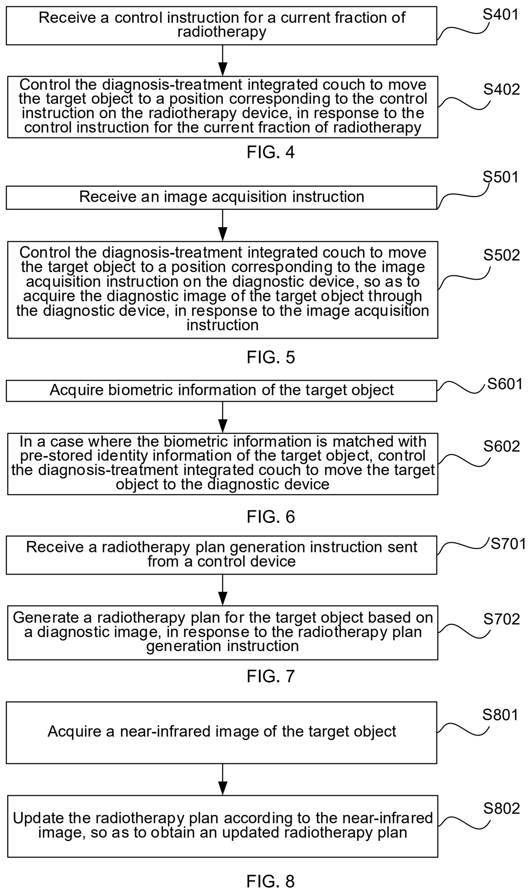

In some embodiments, for disease conditions of some patients (i.e., the target object), a radiotherapy plan with multi-fraction of radiotherapy may be formulated, and therefore, in a case where the target object requires the radiotherapy device for multi-fraction of radiotherapy, the diagnostic image acquired by the diagnostic device may include the diagnostic image in each fraction of radiotherapy. For example, in a current fraction of radiotherapy, the diagnostic device may acquire a diagnostic image for the current fraction of the target object. In this way, when formulating the radiotherapy plan, the radiotherapy plan of each fraction of radiotherapy may be formulated according to the diagnostic image in each fraction of radiotherapy. Correspondingly, the control device needs to adjust the position of the target object on the radiotherapy device according to the radiotherapy plan of each fraction of radiotherapy, so as to enable the radiotherapy device to perform precise radiotherapy on the target object. Therefore, as shown in FIG. 4, the radiotherapy control method provided in the embodiments of the present disclosure also includes:
S401, the control device receives a control instruction for the current fraction of radiotherapy.

The control instruction for the current fraction of radiotherapy includes: a control instruction generated based on a comparison result between the diagnostic image for the current fraction and an initial diagnostic image, or generated based on a comparison result between the diagnostic image for the current fraction and a diagnostic image for a previous fraction.

In some embodiments, the initial diagnostic image is: acquired by the diagnostic device when performing a first fraction of radiotherapy on the target object, or acquired by the diagnostic device when performing an initial diagnosis on the target object.

As can be seen from the above, the radiotherapy plan for the target object may be directly generated by the control device, or generated by the TPS server.

In a case where the radiotherapy plan is directly generated by the control device, the control device may acquire the diagnostic image for the current fraction and the initial diagnostic image from the diagnostic device, and generate the control instruction for the current fraction of radiotherapy based on the comparison result between the diagnostic image for the current fraction and the initial diagnostic image. Alternatively, the control device may acquire the diagnostic image for the current fraction and the diagnostic image for the previous fraction from the diagnostic device, and generate the control instruction for the current fraction of radiotherapy based on the comparison result between the diagnostic image for the current fraction and the diagnostic image for the previous fraction.

In a case where the radiotherapy plan is generated by the TPS server, the TPS server may acquire the diagnostic image for the current fraction and the initial diagnostic image from the diagnostic device, and generate the radiotherapy plan for the current fraction of radiotherapy based on the comparison result between the diagnostic image for the current fraction and the initial diagnostic image. Alternatively, the TPS server may acquire the diagnostic image for the current fraction and the diagnostic image for the previous fraction from the diagnostic device, and generate the radiotherapy plan for the current fraction of radiotherapy based on the comparison result between the diagnostic image for the current fraction and the diagnostic image for the previous fraction. Subsequently, the TPS server generates a control instruction for the current fraction of radiotherapy according to the radiotherapy plan of the current fraction of radiotherapy, and sends the control instruction for the current fraction of radiotherapy to the control device.

S402, the control device controls the diagnosis-treatment integrated couch to move the target object to a position corresponding to the control instruction on the radiotherapy device, in response to the control instruction for the current fraction of radiotherapy.

In some embodiments, in a case where a radiotherapy plan with multi-fraction of radiotherapy is formulated for the target object, the diagnostic device also needs to acquire the diagnostic image in each fraction of radiotherapy. Correspondingly, the control device needs to adjust the position of the target object on the diagnostic device according to the diagnostic image of each fraction of radiotherapy, so as to enable the diagnostic device to acquire the accurate diagnostic image. In this case, as shown in FIG. 5, the method of the control device controlling the diagnosis-treatment integrated couch to move the target object to the diagnostic device, so as to acquire the diagnostic image of the target object through the diagnostic device, specifically includes:
S501, the control device receives an image acquisition instruction.

The image acquisition instruction is generated based on a comparison result between registration points of the diagnostic image for the previous fraction and the initial diagnostic image; the registration points include: a sagittal plane or a coronal plane of a target region of the target object.

In some embodiments, in a case where the control device is integrated with an image comparison function, the control device may directly generate the image acquisition instruction according to the comparison result between the registration points of the diagnostic image for the previous fraction and the initial diagnostic image.

In a case where the control device is not integrated with an image comparison function, the diagnostic device may generate the comparison result according to the comparison result between the registration points of the diagnostic image for the previous fraction and the initial diagnostic image. Subsequently, the diagnostic device may generate an image acquisition instruction according to the comparison result and send the image acquisition instruction to the control device.

S502, the control device controls the diagnosis-treatment integrated couch to move the target object to a position corresponding to the image acquisition instruction on the diagnostic device, so as to acquire the diagnostic image of the target object through the diagnostic device, in response to the image acquisition instruction.

In some embodiments, in order to accurately determine a radiotherapy object as the target object, identity identification may thus be performed for the target object. In this case, as shown in FIG. 6, the method of the control device controlling the diagnosis-treatment integrated couch to move the target object to the diagnostic device, specifically includes:
S601, the control device acquires biometric information of the target object.

In some embodiments, an image acquisition device may be set at the entrance of the space where the diagnostic device is located, or on the diagnostic device. The image acquisition device may acquire the biometric information of the target object. Subsequently, the image acquisition device may send the acquired biometric information of the target object to the control device.

In some embodiments, the above-mentioned biometric information may be an iris feature, a face feature, a fingerprint feature, etc., of the target object.

S602, in a case where the biometric information is matched with pre-stored identity information of the target object, the control device controls the diagnosis-treatment integrated couch to move the target object to the diagnostic device.

That is, the control device may first acquire the biometric information of the target object before controlling the diagnosis-treatment integrated couch to move the target object to the diagnostic device, and match the biometric information with the pre-stored identity information of the target object. Only in a case where the matching is successful, it may control the diagnosis-treatment integrated couch to move the target object to the diagnostic device, so as to avoid incorrect radiotherapy objects.

In some embodiments, the radiotherapy plan generation method provided in the embodiments of the present disclosure is performed by the TPS server 1022 in FIG. 1. FIG. 7 shows a schematic flowchart of a radiotherapy plan generation method provided in the embodiments of the present disclosure. As shown in FIG. 7, the radiotherapy plan generation method includes: S701 to S702.

S701, the TPS server receives a radiotherapy plan generation instruction sent from a control device.

The radiotherapy plan generation instruction is triggered synchronously when the control device controls a diagnosis-treatment integrated couch to move a target object from a diagnostic device to a radiotherapy device.

S702, the TPS server generates a radiotherapy plan for the target object based on a diagnostic image, in response to the radiotherapy plan generation instruction.

The diagnostic image is acquired by the diagnostic device, through the control device controlling the diagnosis-treatment integrated couch to move the target object to the diagnostic device, before triggering the radiotherapy plan generation instruction.

In some embodiments, after the TPS server acquires the diagnostic image of the target object, it may map a position coordinate of the target object in the diagnostic image to the same coordinate system as a position coordinate of the diagnosis-treatment integrated couch, and generate the radiotherapy plan for the target object according to a target point coordinate or a field coordinate of the target object.

In some embodiments, when the diagnosis-treatment integrated couch has a certain attenuation influence on rays of the radiotherapy device, the TPS server, when generating the radiotherapy plan, may invoke 3D model data to simulate and calculate the attenuation influence of the diagnosis-treatment integrated couch on the rays, and then generate a radiotherapy plan including reasonable rays.

In some embodiments, in a case where the target object requires the radiotherapy device to perform multi-fraction of radiotherapy, the diagnostic image includes: a diagnostic image for a current fraction for the target object, acquired by the diagnostic device, in the current fraction of radiotherapy. The method of the TPS server generating the radiotherapy plan for the target object based on the diagnostic image, specifically includes:
comparing the diagnostic image for the current fraction with a diagnostic image for a previous fraction, and optimizing a previous fraction of radiotherapy plan for the target object based on a comparison result between the diagnostic image for the current fraction and the diagnostic image for the previous fraction, so as to obtain a radiotherapy plan for the current fraction for the target object. Alternatively, the diagnostic image for the current fraction is compared with an initial plan image, and an initial radiotherapy plan for the target object is optimized based on a comparison result between the diagnostic image for the current fraction and the initial plan image, so as to obtain a radiotherapy plan for the current fraction for the target object.

In some embodiments, after obtaining the radiotherapy plan for the current fraction for the target object, the TPS server may send a control instruction for the current fraction of radiotherapy to the control device, according to the radiotherapy plan for the current fraction for the target object, so as to enable the control device to control the diagnosis-treatment integrated couch to move the target object to a position corresponding to the control instruction.

The detailed process of the TPS server generating the radiotherapy plan for the target object based on the diagnostic image may be referred to the detailed description in S401 above, which will not be repeated here.

In some embodiments, the target object is usually injected with a near-infrared fluorescent agent. In this way, a near-infrared image of the target object may be acquired, and then, according to the near-infrared image of the target object, the target region may be tracked and the radiotherapy plan may be updated. In this case, as shown in FIG. 8, the radiotherapy plan generation method provided in the embodiments of the present disclosure, after the TPS server generates the radiotherapy plan for the target object based on the diagnostic image, also includes:
S801, the TPS server acquires a near-infrared image of the target object.

In some embodiments, the TPS server may acquire the near-infrared image of the target object from the diagnostic device, or acquire the near-infrared image of the target object from other image acquisition devices, which is not limited in the embodiments of the present disclosure.

S802, the TPS server updates the radiotherapy plan according to the near-infrared image, so as to obtain an updated radiotherapy plan.

In some embodiments, when the TPS server acquires the near-infrared image of the target object from the diagnostic device, the TPS server may directly update the radiotherapy plan according to the near-infrared image, so as to obtain the updated radiotherapy plan.

When the TPS server acquires the near-infrared image of the target object from other image acquisition devices, the TPS server may first map a position of a target region of the target object in the near-infrared image to a position of the target region of the target object in the radiotherapy plan, and then update the radiotherapy plan according to the mapping relationship, so as to obtain the updated radiotherapy plan.

The above mainly introduces the solutions of the embodiments of the present application from the perspective of methods. It can be understood that the control device and the TPS server, in order to implement the above functions, contain the corresponding hardware structures and/or software modules for performing the respective functions. Those skilled in the art should easily realize that, in conjunction with units and algorithmic steps of each example described in the embodiments disclosed herein, the embodiments of the present disclosure can be implemented in the form of hardware or a combination of hardware and computer software. Whether a function is performed by hardware or by computer software driving hardware, depends on the specific applications and design constraints of the technical solutions. Professional technicians may use different methods for each specific application, so as to implement the described functions, but such an implementation should not be regarded as beyond the scope of the embodiments of the present disclosure.

According to the embodiments of the present disclosure, the present disclosure also provides an electronic device, including: at least one processor; and a memory communicatively connected with the at least one processor; where the memory has stored instructions that can be executed by the at least one processor, and the instructions are executed by the at least one processor to enable the at least one processor to perform the radiotherapy control method provided in the present disclosure.

According to the embodiments of the present disclosure, the present disclosure also provides a non-transient computer-readable storage medium storing computer instructions, where the computer instructions are used to enable an electronic device to perform the radiotherapy control method provided in the present disclosure.

According to the embodiments of the present disclosure, the present disclosure also provides a computer program product, including a computer program, where the computer program, when executed by a processor, implements the radiotherapy control method provided in the present disclosure.

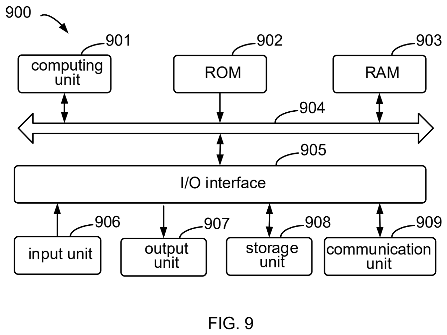

FIG. 9 shows a schematic block diagram of an example electronic device 900 that may be used to implement the embodiments of the present disclosure. The electronic device is intended to represent various forms of digital computers, such as a laptop computer, a desktop computer, a workbench, a personal digital assistant, a server, a blade server, a mainframe server, and other suitable computers. The electronic device may also represent various forms of mobile apparatuses, such as a personal digital assistant, a cellular phone, a smart phone, a wearable device and other similar computing apparatuses. The components, their connections and relationships, as well as their functions shown herein are provided as examples only and are not intended to limit implementations of the present disclosure described and/or claimed herein. In some embodiments, the electronic device may be the radiotherapy control device shown in FIG. 1 above.

As shown in FIG. 9, the electronic device 900 includes a computing unit 901, which may perform various appropriate actions and processes according to a computer program stored in a read-only memory (ROM) 902 or a computer program loaded to a random access memory (RAM) 903 from a storage unit 908. Various programs and data required for the operations of the electronic device 900 may also be stored in the random access memory (RAM) 903. The computing unit 901, the read-only memory 902, and the RAM 903 are connected to each other via a bus 904. An input/output (I/O) interface 905 is also connected to the bus 904.

A plurality of components in the electronic device 900 are connected to the input/output interface 905. The plurality of components include: an input unit 906, such as a keyboard, a mouse, etc.; an output unit 907, such as various types of displays, speakers, etc.; a storage unit 908, such as a magnetic disk, an optical disk, etc.; and a communication unit 909, such as a network card, a modem, a wireless communication transceiver, etc. The communication unit 909 allows the electronic device 900 to exchange information/data with other devices over a computer network and/or various telecommunication networks such as the Internet.

The computing unit 901 may be a variety of general-purpose and/or special-purpose processing components with processing and computing capabilities. Some examples of the computing unit 901 include, but are not limited to, a central processing unit, a graphics processing unit (GPU), various special-purpose artificial intelligence (Al) computing chips, various computing units for executing machine learning model algorithms, a digital signal processor, and any appropriate processor, controller and microcontroller, etc. The computing unit 901 performs the various methods and processes described above, such as the radiotherapy control method and the radiotherapy plan generation method. For example, in an embodiment, the radiotherapy control method and the radiotherapy plan generation method may be implemented as a computer software program, which is tangibly included in a machine-readable medium, such as the storage unit 908. In an embodiment, a part or all of the computer program may be loaded and/or installed onto the electronic device 900 via the ROM 902 and/or the communication unit 909. When the computer program is loaded onto the RAM 903 and executed by the computing unit 901, one or more steps of the radiotherapy control method and the radiotherapy plan generation method described above may be performed. Alternatively, in other embodiments, the computing unit 901 may be configured to execute the radiotherapy control method and the radiotherapy plan generation method in any other appropriate manners (e.g., by means of firmware).

The various implementations of systems and techniques described above herein may be implemented in a digital electronic circuitry system, an integrated circuitry system, a field-programmable gate array, an application-specific integrated circuit, an application-specific standard product (Application Specific Standard Part, ASSP), a system on a chip (SOC) system, a complex programmable logic device (CPLD), computer hardware, firmware, software and/or any combination thereof. The various implementations may include implementations in one or more computer programs. The one or more computer programs may be executed and/or interpreted on a programmable system including at least one programmable processor. The programmable processor may be a special-purpose or general-purpose programmable processor, may receive data and instructions from a storage system, at least one input apparatus and at least one output apparatus, and transmit the data and instructions to the storage system, the at least one input apparatus and the at least one output apparatus.

Program codes for the implementation of the method of the present disclosure may be written in any combination of one or more programming languages. These program codes may be provided for a processor or controller of a general-purpose computer, a special-purpose computer or other programmable data processing apparatuses, to cause functions/operations specified in the flowcharts and/or block diagrams to be implemented when the program codes are executed by the processor or controller. The program codes may all be executed on a machine, or partially be executed on a machine, or partially be executed on a machine and partially be executed on a remote machine as a separate software package, or all be executed on a remote machine or a server.

In the context of the present disclosure, the machine-readable medium may be a tangible medium that contains or stores a program used for an instruction execution system, apparatus or device, or a program used in conjunction with an instruction execution system, apparatus or device. The machine-readable media may be machine-readable signal media or machine-readable storage media. The machine-readable medium may include, but be not limited to, an electronic, magnetic, optical, electromagnetic, infrared or semi-conductive system, apparatus or device, or any appropriate combination thereof. More specific examples of the machine-readable storage medium may include an electrical connection based on one or more wires, a portable computer disk, a hard disk, a random-access memory, a read-only memory, an erasable programmable read-only memory, an optical fiber, a portable compact disc read-only memory, an optical storage device, a magnetic storage device, or any appropriate combination thereof.

In order to provide interactions with a user, the systems and techniques described herein may be implemented on a computer. The computer has: a display apparatus (for example, a cathode ray tube (CRT) or liquid crystal display (LCD) monitor) for displaying information to the user; and a keyboard and a pointing apparatus (for example, a mouse or a trackball), by which the user may provide an input to the computer. Other types of apparatuses may also be used to provide interactions with the user. For example, feedback provided for the user may be sensory feedback in any form (for example, visual feedback, auditory feedback or haptic feedback). Moreover, an input (including sound input, voice input or haptic input) from the user may be received in any form.

The systems and techniques described herein may be implemented in a computing system including a back-end component (for example, as a data server), a computing system including a middleware component (for example, an application server), a computing system including a front-end component (for example, a client computer with a graphical user interface or a web browser, by which the user may interact with implementations of the systems and techniques described herein), or a computing system including any combination of such a back-end component, middleware component or front-end component. The components of the system may be interconnected by any form or medium of digital data communication (for example, a communication network). Examples of the communication network include: a local area network (LAN), a wide area network (WAN) and the Internet.

The computing system may include a client and a server. The client and the server are generally remote from each other and typically interact via a communication network. A relationship between the client and the server is generated by computer programs that are running on the respective computers and have a client-server relationship with each other. The server may be a cloud server, a server of a distributed system, or a server combined with blockchains.

It should be understood that, the various forms of flows shown above may be used, with steps reordered, added or removed. For example, the various steps recorded in the present disclosure may be executed in parallel, in sequence or in a different order, as long as a desired result of the technical solutions of the present disclosure is achieved, which is not limited herein.

The above specific implementations do not constitute a limitation on the protection scope of the present disclosure. It should be understood by those skilled in the art that various modifications, combinations, sub-combinations and substitutions may be made according to design requirements and other factors. Any modifications, equivalent substitutions, improvements and the like made within the spirit and principle of the present disclosure shall fall within the protection scope of the present disclosure.

## Claims

1. A radiotherapy control method, **characterized in that** the method is performed by a control device, and comprises:
controlling a diagnosis-treatment integrated couch to move a target object to a diagnostic device, so as to acquire a diagnostic image of the target object through the diagnostic device; and
controlling the diagnosis-treatment integrated couch to move the target object from the diagnostic device to a radiotherapy device, and synchronously triggering generation of a radiotherapy plan for the target object based on the diagnostic image.

2. The method according to claim 1, wherein controlling the diagnosis-treatment integrated couch to move the target object from the diagnostic device to the radiotherapy device, and synchronously triggering the generation of the radiotherapy plan for the target object based on the diagnostic image, comprises:
controlling the diagnosis-treatment integrated couch to move the target object from the diagnostic device to the radiotherapy device, and synchronously triggering the generation of the radiotherapy plan for the target object based on the diagnostic image during the movement of the diagnosis-treatment integrated couch; or,
synchronously triggering the generation of the radiotherapy plan for the target object based on the diagnostic image at a beginning of the diagnosis-treatment integrated couch to move the target object from the diagnostic device to the radiotherapy device; or,
synchronously triggering the generation of the radiotherapy plan for the target object based on the diagnostic image at a moment when the diagnostic image is acquired, and controlling the diagnosis-treatment integrated couch to move the target object from the diagnostic device to the radiotherapy device after the diagnostic device acquires the diagnostic image.

3. The method according to claim 1, wherein triggering the generation of a radiotherapy plan for the target object based on the diagnostic image, comprises:
in a case where the control device is integrated with a radiotherapy plan generation function, triggering the control device to generate the radiotherapy plan for the target object based on the diagnostic image; or,
in a case where the control device is not integrated with a radiotherapy plan generation function, sending a radiotherapy plan generation instruction to a radiation treatment planning system (TPS) server, so as to trigger the TPS server to generate the radiotherapy plan for the target object based on the diagnostic image.

4. The method according to claim 1, wherein when the diagnosis-treatment integrated couch is controlled to move the target object from the diagnostic device to the radiotherapy device, the radiotherapy plan for the target object has been generated.

5. The method according to claim 4, further comprising:
acquiring a predicted generation duration of the radiotherapy plan; and
determining a moving speed of the diagnosis-treatment integrated couch according to the predicted generation duration, so as to enable that the radiotherapy plan for the target object has been generated when the diagnosis-treatment integrated couch moves the target object from the diagnostic device to the radiotherapy device.

6. The method according to claim 5, wherein the predicted generation duration is predicted according to a generation duration of a radiotherapy plan for a reference image; and the reference image is an image whose similarity to the diagnostic image is greater than a preset similarity.

7. The method according to claim 1, wherein in a case where the target object requires the radiotherapy device to perform multi-fraction of radiotherapy, the diagnostic image comprises: a diagnostic image for a current fraction for the target object, acquired by the diagnostic device, in the current fraction of radiotherapy; the method further comprises:
receiving a control instruction for the current fraction of radiotherapy; the control instruction for the current fraction of radiotherapy comprises: a control instruction generated based on a comparison result between the diagnostic image for the current fraction and an initial diagnostic image, or generated based on a comparison result between the diagnostic image for the current fraction and a diagnostic image for a previous fraction;
in response to the control instruction for the current fraction of radiotherapy, controlling the diagnosis-treatment integrated couch to move the target object to a position corresponding to the control instruction on the radiotherapy device.

8. The method according to claim 7, wherein controlling the diagnosis-treatment integrated couch to move the target object to the diagnostic device, so as to acquire the diagnostic image of the target object through the diagnostic device, comprises:
receiving an image acquisition instruction; the image acquisition instruction is generated based on a comparison result between registration points of the diagnostic image for the previous fraction and the initial diagnostic image; the registration points comprise: a sagittal plane or a coronal plane of a target region of the target object; and
in response to the image acquisition instruction, controlling the diagnosis-treatment integrated couch to move the target object to a position corresponding to the image acquisition instruction on the diagnostic device, so as to acquire the diagnostic image of the target object through the diagnostic device;
wherein the initial diagnostic image is: acquired by the diagnostic device when performing a first fraction of radiotherapy on the target object, or acquired by the diagnostic device when performing an initial diagnosis on the target object.

9. The method according to claim 1, wherein controlling the diagnosis-treatment integrated couch to move the target object to the diagnostic device, comprises:
acquiring biometric information of the target object; and
in a case where the biometric information is matched with pre-stored identity information of the target object, controlling the diagnosis-treatment integrated couch to move the target object to the diagnostic device.

10. A radiotherapy plan generation method, **characterized in that** the method is performed by a radiation treatment planning system (TPS) server, and comprises:
receiving a radiotherapy plan generation instruction sent from a control device; the radiotherapy plan generation instruction is triggered synchronously when the control device controls a diagnosis-treatment integrated couch to move a target object from a diagnostic device to a radiotherapy device; and
in response to the radiotherapy plan generation instruction, generating a radiotherapy plan for the target object based on a diagnostic image; the diagnostic image is acquired by the diagnostic device, through the control device controlling the diagnosis-treatment integrated couch to move the target object to the diagnostic device, before triggering the radiotherapy plan generation instruction.

11. The method according to claim 10, wherein in a case where the target object requires the radiotherapy device to perform multi-fraction of radiotherapy, the diagnostic image comprises: a diagnostic image for a current fraction for the target object, acquired by the diagnostic device, in the current fraction of radiotherapy; generating the radiotherapy plan for the target object based on the diagnostic image, comprises:
comparing the diagnostic image for the current fraction with a diagnostic image for a previous fraction, and optimizing a previous fraction of radiotherapy plan for the target object based on a comparison result between the diagnostic image for the current fraction and the diagnostic image for the previous fraction, so as to obtain a radiotherapy plan for the current fraction for the target object; or
comparing the diagnostic image for the current fraction with an initial plan image, and optimizing an initial radiotherapy plan for the target object based on a comparison result between the diagnostic image for the current fraction and the initial plan image, so as to obtain a radiotherapy plan for the current fraction for the target object.

12. The method according to claim 11, further comprising:
sending a control instruction for the current fraction of radiotherapy to the control device, according to the radiotherapy plan for the current fraction for the target object, so as to enable the control device to control the diagnosis-treatment integrated couch to move the target object to a position corresponding to the control instruction.

13. The method according to claim 10, wherein after generating the radiotherapy plan for the target object based on the diagnostic image, the method further comprises:
acquiring a near-infrared image of the target object; and
updating the radiotherapy plan according to the near-infrared image, so as to obtain an updated radiotherapy plan.

14. An electronic device, **characterized in that** the electronic device comprises:
a processor;
a memory, configured to store instructions executable by the processor;
wherein the processor is configured to execute the instructions to implement the method according to any one of claims 1 to 9, or the method according to any one of claims 10 to 13.

15. A radiotherapy system, **characterized by** comprising:
a control device, configured to perform the method according to any one of claims 1 to 9; and
a TPS server, configured to perform the method according to any one of claims 10 to 13.
